(19) **Europäisches Patentamt**
**European Patent Office**
**Office européen des brevets**

(11) **EP 2 311 524 B1**

(12) **FASCICULE DE BREVET EUROPEEN**

(45) Date de publication et mention
de la délivrance du brevet:
**22.06.2011 Bulletin 2011/25**

(51) Int Cl.:
**A61N 1/368** (2006.01)

(21) Numéro de dépôt: **10168396.9**

(22) Date de dépôt: **05.07.2010**

(54) **Dispositif médical implantable actif de resynchronisation cardiaque à optimisation automatique des délais interventriculaire et atrioventriculaire**

Implantierbarer medizinischer Herzschrittmacher und Verfahren zur Optimierung des Implantatortes für interventrikuläre und atrioventrikuläre Intervalle

Medical implantable cardiac pacemaker with automatic interventricular and atrioventricular intervals optimization

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **19.10.2009 FR 0957316**

(43) Date de publication de la demande:
**20.04.2011 Bulletin 2011/16**

(73) Titulaire: **Sorin CRM SAS**
**92541 Montrouge (FR)**

(72) Inventeurs:
• **Renesto, Fabrizio**
**10013, Borgofranco d'Ivrea (TO) (IT)**

• **Casset, Cyrille**
**33650, SAINT SELVE (FR)**

(74) Mandataire: **Dupuis-Latour, Dominique**
**SEP Bardehle Pagenberg Dost Altenburg Geissler**
**10, boulevard Haussmann**
**75009 Paris (FR)**

(56) Documents cités:
**EP-A1- 1 977 784      EP-A1- 2 092 885**
**WO-A1-2005/089866    US-A1- 2005 027 320**

## Description

L'invention concerne les "dispositifs médicaux implantables actifs" tels que définis par la directive 90/385/CEE du 20 juin 1990 du Conseil des communautés européennes.

Elle concerne plus précisément les implants permettant de surveiller en continu le rythme cardiaque et délivrer si nécessaire au coeur des impulsions électriques permettant d'assurer une stimulation conjointe et permanente des ventricules gauche et droit afin de resynchroniser ces derniers, technique dite "CRT" (*Cardiac Resynchronization Therapy*) ou "BVP" (*BiVentricular Pacing*).

À cet effet, on implante au patient un appareil muni d'électrodes permettant de stimuler l'un et l'autre ventricule. Le dispositif est capable de surveiller en continu le rythme cardiaque et délivrer si nécessaire au coeur des impulsions électriques pour stimuler conjointement les ventricules gauche et droit afin de resynchroniser ces derniers.

Le dispositif applique entre les instants respectifs de stimulation des ventricules gauche et droit un délai dit "délai interventriculaire" (DVV ou VVD) ajusté de manière à resynchroniser la contraction des deux ventricules pour optimiser l'état hémodynamique du patient.

Le US 2005/0027320 A1 propose ainsi d'analyser le signal délivré par un capteur de mouvement de la paroi interventriculaire pour ajuster le DVV à une valeur optimale.

Un autre stimulateur CRT est divulgué dans le EP 1 108 446 A1 (ELA Medical), qui décrit un dispositif permettant d'appliquer entre les deux stimulations ventriculaires un DVV variable, ajusté de manière à resynchroniser la contraction des ventricules avec optimisation fine de l'état hémodynamique du patient.

En effet, une stimulation simultanée des deux ventricules n'est pas toujours optimale, car elle n'aboutit pas forcément à une contraction synchrone des deux ventricules du fait, d'une part, des délais de conduction au sein du myocarde qui ne sont pas les mêmes à droite et à gauche et peuvent dépendre de multiples facteurs et, d'autre part, de l'emplacement de la sonde ventriculaire gauche, selon que celle-ci est une sonde enfilée dans le sinus coronaire ou une sonde épicardique. Il est donc souhaitable d'établir un délai entre les deux stimulations, et d'ajuster ce délai pour resynchroniser la contraction des ventricules et assurer ainsi une optimisation fine de l'hémodynamique. Le DVV pourra être nul, positif (le ventricule gauche étant stimulé après le ventricule droit), ou négatif (le ventricule droit étant stimulé après le ventricule gauche).

Les dispositifs CRT incluent en outre un mode de fonctionnement "double chambre" classique dans lequel le dispositif surveille l'activité ventriculaire après un événement auriculaire spontané (détection d'une onde P de dépolarisation auriculaire) ou stimulé (application d'une impulsion A de stimulation auriculaire). En même temps, le dispositif commence à compter un délai dit "délai atrioventriculaire" (DAV ou AVD) tel que si aucune activité spontanée ventriculaire (onde R) n'a été détectée à l'issue de ce délai, alors le dispositif déclenche une stimulation de ce ventricule (application d'une impulsion V).

Cette thérapie de resynchronisation basée sur une stimulation conjointe des deux ventricules requiert la sélection d'un certain nombre de paramètres. Ces paramètres doivent être adaptés à chaque patient afin de remédier aux troubles (spontanés ou induits par une stimulation traditionnelle) de la contraction myocardique, tels que dilatation des cavités cardiaques, faible fraction d'éjection, allongement excessif de la durée du complexe QRS.

Des études cliniques ont permis d'observer des résultats souvent spectaculaires pour des patients en insuffisance cardiaque non améliorée par les traitements classiques, dès lors que les paramètres de la thérapie CRT ont été ajustés de façon précise en fonction du patient et de la nature de son trouble.

L'ensemble de ces paramètres sera désigné par "configuration de stimulation", terme regroupant les caractéristiques relatives aux sites de stimulation (c'est-à-dire à l'emplacement physique des électrodes et au choix des électrodes, dans le cas des dispositifs multisite) et celles relatives à la séquence de stimulation (c'est-à-dire à l'ordre suivant lequel les impulsions de stimulation sont appliquées au coeur sur les différents sites choisis, et les intervalles de temps séparant l'application de ces impulsions successives).

Il est en outre nécessaire de réévaluer ultérieurement ces réglages pour les réajuster éventuellement. En effet, les effets bénéfiques procurés par la thérapie CRT peuvent conduire, à terme, à modifier la configuration primitive et le paramétrage de la stimulation.

La technique de référence pour l'ajustement des paramètres de stimulation CRT est l'évaluation par échocardiographie avec estimation des délais caractéristiques de la systole, en particulier le temps d'ouverture de la valve aortique. Cette procédure, qui doit être mise en oeuvre en milieu hospitalier et par un personnel qualifié, est longue et coûteuse et ne peut pas être appliquée aussi souvent que cela serait utile ou nécessaire sans interférer avec la vie quotidienne du patient, malgré les effets bénéfiques de la thérapie CRT.

Une autre difficulté inhérente à l'évaluation échocardiographique tient au fait qu'elle nécessite de tester successivement plusieurs configurations de stimulation, en déterminant pour chacune un DAV optimal. Le nombre de combinaisons à tester est de ce fait important, et implique une procédure lourde, longue et difficile à gérer, ce qui exclut d'en faire une opération de routine.

Au surplus, même avec une mise en oeuvre complète de ces procédures, il reste environ 30 % de patients qui ne répondent pas à la thérapie CRT, avec les conséquences graves que l'on peut imaginer en termes de qualité de vie, d'hospitalisations liées à l'insuffisance cardiaque et de réduction de l'espérance de vie.

La plupart des études se concentrent aujourd'hui sur la manière de traiter cette population de patients réfractaires par expérimentation de nouvelles configurations de stimulation, et en cherchant à optimiser le paramétrage de la stimulation, aussi bien lors de l'implantation que de façon suivie, par des réévaluations périodiques.

Il existe de ce fait un besoin réel d'une technique permettant d'évaluer de façon simple, rapide, automatisée et précise l'incidence des différents paramètres de la thérapie CRT, notamment des délais DAV et DVV, de manière à pouvoir optimiser l'état hémodynamique du patient.

Le EP 1 736 203 A1 (ELA Medical) décrit un dispositif CRT qui utilise à cet effet les paramètres liés à l'accélération endocardiaque (EA) pour déterminer une configuration de stimulation optimale, au moment de l'implantation ou ultérieurement. L'accélération endocardiaque est par exemple mesurée par un accéléromètre intégré dans une sonde endocavitaire, comme cela est par exemple décrit dans le EP 0 515 319 A1 (Sorin Biomedica Cardio SpA)

En effet, diverses études cliniques ont montré que l'accélération endocardiaque est un paramètre qui reflète très précisément et en temps réel les phénomènes liés aux mouvements de la cavité cardiaque, et permet de ce fait de fournir des informations très complètes sur la mécanique cardiaque, aussi bien dans le cas d'un fonctionnement normal que d'un fonctionnement déficient.

Plus précisément, le EP 1 736 203 A1 précité propose d'établir une caractéristique PEA vs. DAV pour chaque configuration de stimulation sélectionnée, en exécutant un balayage du délai atrioventriculaire DAV et en enregistrant les variations de l'amplitude du pic d'accélération endocardiaque (PEA), généralement le premier pic PEA1. Cette caractéristique est déterminée périodiquement dans un mode de test déclenché par l'implant, et les résultats obtenus sont traités et combinés pour donner un indice de performance composite reflétant l'efficacité de la configuration choisie, et permettre ainsi le choix d'une configuration maximisant cet indice, considérée comme optimale. Cet indice de performance est notamment dérivé de l'aire sous la caractéristique PEA vs. DAV, censée refléter l'efficacité de la fonction ventriculaire.

Une autre méthode d'optimisation est décrite dans l'article de JM Dupuis et al. : Programming Optimal Atrioventricular Delay in Dual Chamber Pacing Using Peak Endocardial Acceleration : Comparison with a Standard Echocardiographic Procedure, PACE 2003; 26: [Pt. II], 210-213. Cette technique consiste à exécuter, ici encore, un balayage du DAV dans la configuration de stimulation considérée pour tracer une caractéristique PEA vs. DAV, mais la valeur considérée optimale du DAV est celle du point d'inflexion de la caractéristique, point correspondant à une durée de remplissage maximale du ventricule sans troncature de l'onde A (retard minimal entre la fermeture de la valve mitrale et le début du complexe QRS). L'algorithme correspondant, s'il donne des résultats satisfaisants, requiert toutefois plusieurs minutes pour son exécution en raison des multiples balayages de DAV pour les diverses valeurs de DVV testées séparément, avant de pouvoir sélectionner le couple {DAV, DVV} optimal. Une autre technique encore, décrite dans le US 2007/0179542 A1 (Medtronic, Inc.) consiste à rechercher une corrélation ou une synchronisation entre les morphologies respectives des signaux EA et EGM (électrogramme endocavitaire), c'est-à-dire entre des signaux reflétant respectivement les paramètres mécaniques (EA) et électriques (EGM). L'analyse de ces signaux donne une valeur supposée optimale du DAV, pour une configuration de stimulation donnée. Mais si l'on souhaite également optimiser le paramètre DVV, il est nécessaire de tester successivement autant de configurations différentes, et d'analyser ensuite les résultats obtenus pour choisir l'une de ces configurations.

Le but de l'invention est de remédier aux inconvénients exposés ci-dessus, en proposant une nouvelle procédure simple, rapide, automatisée et précise pour optimiser concomitamment les deux paramètres DAV et DVV, et ce malgré le caractère interdépendant de ces deux paramètres. On verra en particulier que la technique de l'invention permet de s'affranchir de l'étape de balayage du DAV pour chacune des configurations de stimulation possible (donc pour chacun des DVV possibles), procédure par nature longue compte tenu du nombre élevé de cycles cardiaques nécessaires pour recueillir un nombre suffisant de points de mesure.

Le point de départ de l'invention consiste à exploiter conjointement deux caractéristiques différentes d'un même signal EA pour optimiser le couple de paramètres {DAV, DVV}.

En effet, comme on l'expliquera plus bas, le signal EA forme au cours d'un même cycle cardiaque plusieurs pics correspondant aux principaux bruits qu'il est possible de reconnaître dans chaque cycle d'un coeur sain. La présente invention repose sur l'analyse concomitante de (i) la composante, dite EA1, liée au premier pic d'accélération endocardiaque PEA1 et étroitement corrélée aux variations de la pression dans le ventricule, et (ii) de la composante d'amplitude beaucoup plus faible, dite EA4, liée au quatrième pic d'accélération endocardiaque PEA4 et directement corrélée à la contraction auriculaire. Cette analyse conduira, essentiellement :

- à considérer l'optimisation du DAV comme une optimisation de l'instant de survenue de la composante EA4, afin que la stimulation ventriculaire puisse intervenir le plus tard possible sans troncature de l'onde A, et
- à considérer l'optimisation du DVV comme une optimisation de l'ampleur de la composante EA1 (représentée notamment par l'amplitude du pic ou par l'énergie de la composante EA1), les deux stimulations ventriculaires étant délivrées avec l'intervalle (positif ou négatif) procurant le volume d'éjection le plus élevé.

En d'autres termes, l'invention propose de combiner :

(i) la mesure d'un paramètre temporel (instant de survenue) lié à la composante EA4 avec
(ii) la mesure d'un paramètre non temporel (amplitude ou énergie) lié à la composante EA1,

pour déterminer le meilleur couple {DAV, DVV}, ceci en tenant compte du fait qu'il existe pour chaque configuration de stimulation, donc pour chaque DVV, une seule valeur optimale du DAV, valeur qui est elle-même dépendante de la configuration sélectionnée.

Plus précisément, l'invention propose un dispositif du type général divulgué par le EP 1 736 203 A1 ou le US 2005/0027320 A1 précités, c'est-à-dire comprenant : des moyens de détection d'événements auriculaires et ventriculaires; des moyens de stimulation des ventricules droit et gauche ; des moyens de calcul d'un délai atrio-ventriculaire DAV, compté à partir de la détection d'un événement auriculaire spontané ou stimulé et à l'issue duquel une stimulation du ventricule droit est appliquée en l'absence d'événement ventriculaire spontané détecté ; des moyens de calcul d'un délai inter-ventriculaire DVV à appliquer entre les instants respectifs de stimulation des ventricules droit et gauche ; et des moyens de recueil d'un signal d'accélération endocardiaque EA représentatif des mouvements produits par les contractions cycliques des cavités auriculaire et ventriculaire.

De façon caractéristique de l'invention, les moyens de calcul du DAV et du DVV comprennent : des moyens pour reconnaître et isoler dans le signal EA une composante EA4 correspondant au quatrième pic d'accélération endocardiaque associé à l'activité auriculaire, et à mesurer un paramètre temporel lié à l'intervalle de temps entre la détection d'un événement auriculaire et un marqueur lié à la composante EA4 immédiatement consécutive ; des moyens pour reconnaître et isoler dans le signal EA une composante EA1 correspondant au premier pic d'accélération endocardiaque associé à la contraction ventriculaire isovolumique, et à mesurer un paramètre non temporel lié à l'amplitude du pic, ou à l'énergie du signal, de la composante EA1 ; des moyens pour déterminer un DAV optimal en fonction dudit paramètre temporel lié à la composante EA4, ce DAV optimal étant calculé pour chaque valeur d'une pluralité de valeurs différentes de DVV, de manière à obtenir ainsi une pluralité de couples de valeurs {DAV optimal, DVV} ; et des moyens pour sélectionner, parmi ladite pluralité de couples de valeurs {DAV optimal, DVV}, l'un de ces couples de valeurs en fonction dudit paramètre non temporel lié à la composante EA1.

Les moyens pour sélectionner l'un des couples de valeurs {DAV optimal, DVV} peuvent être alors des moyens pour sélectionner celui des couples qui maximise l'amplitude du premier pic d'accélération endocardiaque ou, respectivement, l'énergie de la composante EA1.

Les moyens pour déterminer le DAV optimal peuvent être notamment des moyens aptes à calculer ce DAV optimal à partir dudit intervalle de temps, augmenté d'une constante de compensation de délai.

Le marqueur lié à la composante EA4 peut être un marqueur de l'instant de survenue du quatrième pic d'accélération endocardiaque, ou bien un marqueur de l'instant de début ou de fin de la composante EA4.

Dans une mode de mise en oeuvre particulier, les moyens pour déterminer le DAV optimal sont des moyens aptes, pour chaque valeur de ladite pluralité de valeurs différentes de DVV : sur un premier cycle cardiaque, à sélectionner au moins une valeur prédéterminée de DAV, mesurer ledit paramètre temporel lié à la composante EA4, et déterminer une valeur correspondante de DAV optimal ; puis, sur le cycle cardiaque immédiatement consécutif, sélectionner une valeur de DAV correspondant à la valeur de DAV optimal déterminée au cycle précédent, et mesurer ledit paramètre non temporel lié à la composante EA1.

La valeur prédéterminée de DAV peut notamment être une valeur longue, choisie de manière à assurer une dépolarisation ventriculaire spontanée consécutive.

Une pluralité de valeurs prédéterminées de DAV peuvent être sélectionnées au cours de cycles successifs, le paramètre temporel lié à la composante EA4 étant mesuré avec une valeur de DAV pour laquelle la composante EA4 peut être reconnue et isolée.

On va maintenant décrire un exemple de mise en oeuvre du dispositif de l'invention, en référence aux dessins annexés où les mêmes références numériques désignent d'une figure à l'autre des éléments identiques ou fonctionnellement semblables.

La Figure 1 est un chronogramme montrant les variations de l'accélération endocavitaire EA au cours de trois cycles cardiaques successifs.

La Figure 2 illustre les différents signaux caractérisant l'activité du coeur au cours d'un cycle cardiaque, avec un relevé d'électrocardiogramme de surface ECG et les variations correspondantes du signal d'accélération endocardiaque EA.

La Figure 3 présente trois exemples de chronogrammes d'un signal EA obtenus, respectivement, dans le cas d'un DAV trop long, d'un DAV correctement ajusté et d'un DAV trop court.

La Figure 4 est un chronogramme d'un signal EA avec divers repères temporels permettant d'illustrer les paramètres analysés avec la technique de l'invention.

La Figure 5 est un organigramme montrant les diverses étapes successives mises en oeuvre par la technique de l'invention.

Les Figures 6a et 6b sont des arbres de séquencement des différentes itérations de l'algorithme d'analyse, respectivement pour les dispositifs connus et pour un dispositif selon la présente invention.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.

L'invention peut notamment être appliquée aux dispositifs implantables tels que ceux de la famille *Paradym CRT* produits et commercialisés par ELA Médical, Montrouge, France (Sorin Group).

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

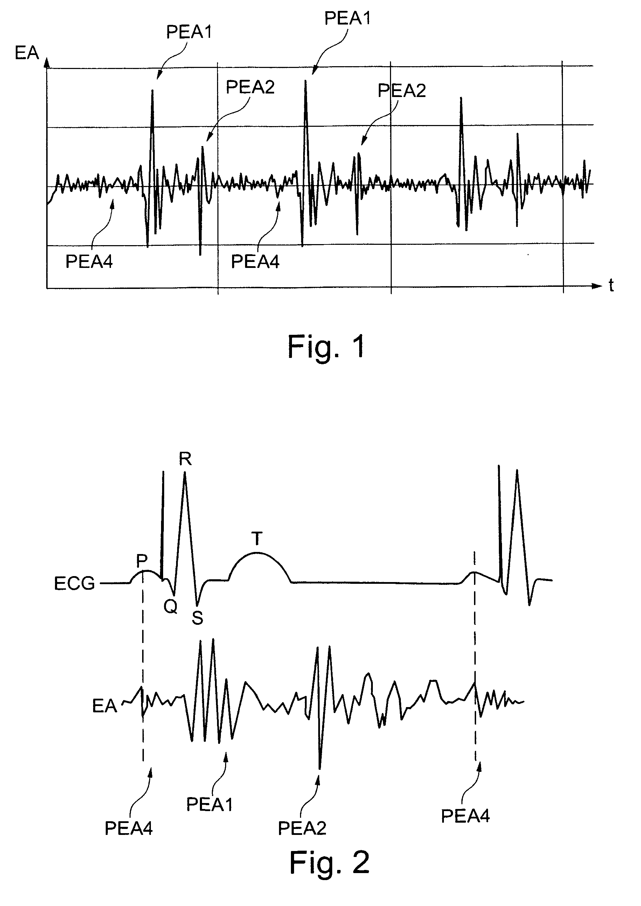

La Figure 1 illustre un exemple de signal d'accélération endocardiaque (EA) recueilli au cours de trois cycles cardiaques successifs.

Le signal EA forme au cours d'un cycle cardiaque deux pics principaux, correspondant aux deux bruits majeurs (sons S1 et S2 du phonocardiogramme) qu'il est possible de reconnaître dans chaque cycle d'un coeur sain :

- le premier pic d'accélération endocardiaque PEA1, dont les variations sont étroitement liées aux variations de la pression dans le ventricule (l'amplitude du pic PEA1 étant, plus précisément, corrélée au maximum positif de la variation de pression dP/dt dans le ventricule gauche) ; et
- le second pic d'accélération endocardiaque PEA2, quant à lui, correspond à la phase de relaxation ventriculaire isovolumique et il est produit par la décélération brusque de la masse sanguine en mouvement dans l'aorte.

Les composantes EA1 et EA2 du signal EA sont celles qui correspondent aux deux pics d'accélération endocardiaque respectifs PEA1 et PEA2.

Le signal EA contient également deux autres composantes, d'amplitude très inférieure, appelées EA3 et EA4, correspondant aux sons S3 et S4 du phonocardiogramme.

La composante EA4, qui est directement liée à la présence d'une contraction auriculaire, présente en particulier un pic PEA4 qui, comme on peut le voir sur la Figure 1, se situe immédiatement avant le pic PEA1. Pour cette dernière raison, ce pic est d'ailleurs quelquefois désigné "PEAO" par les rythmologues, dans la mesure où, du point de vue électrique, la contraction de l'oreillette précède la contraction du ventricule ; en revanche, si l'on se place du point de vue du flux sanguin pompé par le myocarde, la contraction de l'oreillette (correspondant à la composante EA4) achève le remplissage du ventricule en fin de diastole (composante EA2) et se situe donc, du point de vue de l'hémodynamique cardiaque, après cette dernière - d'où la désignation "PEA4".

La Figure 2 illustre les différents signaux caractérisant l'activité du coeur au cours d'un cycle cardiaque donné, avec un relevé d'électrocardiogramme de surface ECG et les variations du signal d'accélération endocardiaque EA.

Le signal ECG comprend successivement : l'onde P correspondant à la dépolarisation de l'oreillette, le complexe QRS correspondant à la dépolarisation du ventricule et l'onde T de repolarisation ventriculaire.

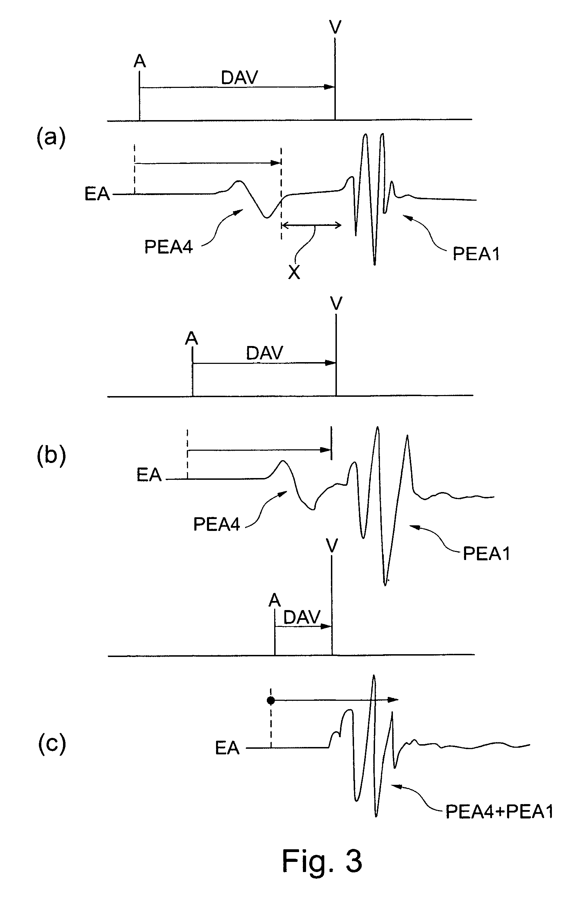

Le signal d'accélération endocardiaque EA, quant à lui, peut se décomposer de la manière suivante : la composante EA4 correspond à la contraction de l'oreillette (onde P), et elle est suivie par la composante EA1, qui débute à la suite du complexe QRS et est engendrée par une combinaison de la fermeture des valves atrio-ventriculaires, de l'ouverture des valves semi-lunaires et de la contraction du ventricule gauche. La composante EA2 qui lui fait suite, quant à elle, accompagne la fin de la systole ventriculaire et elle est générée par la fermeture des valves semi-lunaires. La Figure 3 illustre l'importance qu'il y a à définir de façon précise la durée du DAV.

En effet, du point de vue de la mécanique cardiaque, le DAV doit être suffisamment long pour permettre à l'oreillette de se contracter complètement et par là même de vider dans le ventricule le sang qu'elle contient, de sorte que la contraction ventriculaire doit se produire après que la contraction atriale ait entièrement eu lieu. Mais elle ne doit pas survenir trop longtemps après, car un DAV trop long risquerait de dissocier le système oreillette/ventricule, avec risque de déclencher des arythmies par conduction rétrograde, ou de réduire l'efficacité hémodynamique du cycle cardiaque : en effet, comme la contraction auriculaire termine le remplissage ventriculaire, le délai entre la fin de ce remplissage et le début de la vidange du ventricule constitue un temps mort, "perdu" sur le plan hémodynamique, outre le fait que tout délai prolongé de DAV impacte sur la diastole ventriculaire (remplissage du ventricule post-vidange) et donc "recule" d'autant la fin de ce remplissage ventriculaire, qui se superpose alors à la contraction atriale suivante.

Il est donc important d'adapter le DAV au mieux pour chaque patient, de manière que le début de la vidange ventriculaire (provoquée par la stimulation du ventricule) intervienne tout de suite après la fin du remplissage du ventricule par l'oreillette.

Ainsi, la Figure 3 présente trois exemples de chronogrammes d'un signal EA obtenu, respectivement, dans le cas d'un DAV trop long, d'un DAV correctement ajusté et d'un DAV trop court :

- la Figure 3a illustre l'incidence d'un DAV trop long, qui laisse subsister un temps mort X entre la fin de la contraction de l'oreillette (révélée par le PEA4) et le début de contraction du ventricule (résultant de la stimulation V, qui produit sur le signal EA le pic PEA1) ;
- la Figure 3b illustre le cas où le DAV a été ajusté à une valeur optimale, permettant de neutraliser le temps mort X pour déclencher la contraction du ventricule (stimulation V) dès la fin du remplissage de celui-ci, c'est-à-dire dès la fin de la contraction atriale - correspondant sur le signal EA à la fin du pic PEA4 ;
- la Figure 3c illustre une situation où le DAV appliqué présente une valeur trop courte : dans ce cas, le ventricule commence à se contracter avant d'avoir été complètement rempli, diminuant de ce fait le volume éjecté, et donc le débit, de la pompe cardiaque. Sur le signal EA, cette situation est révélée par une fusion du pic PEA4 et du pic PEA1 immédiatement consécutif.

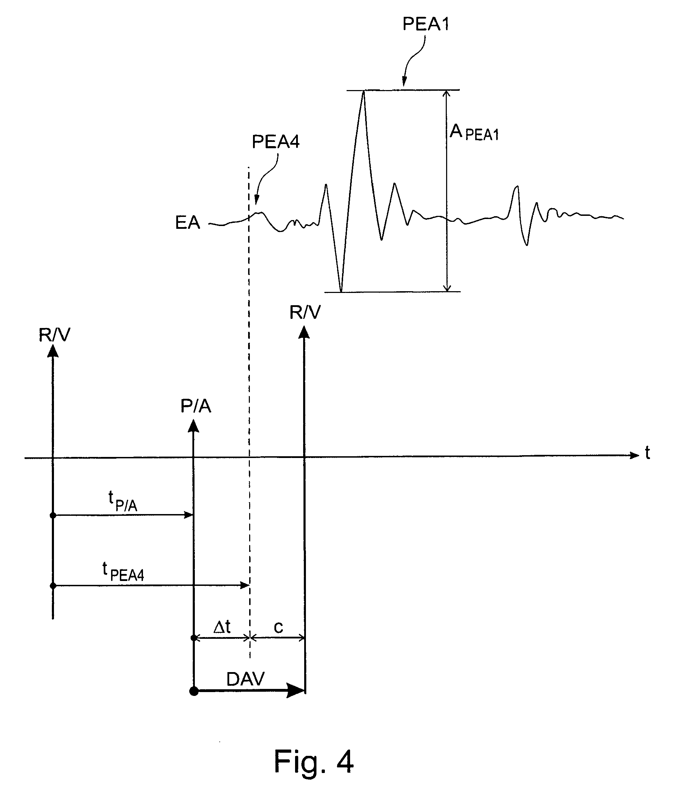

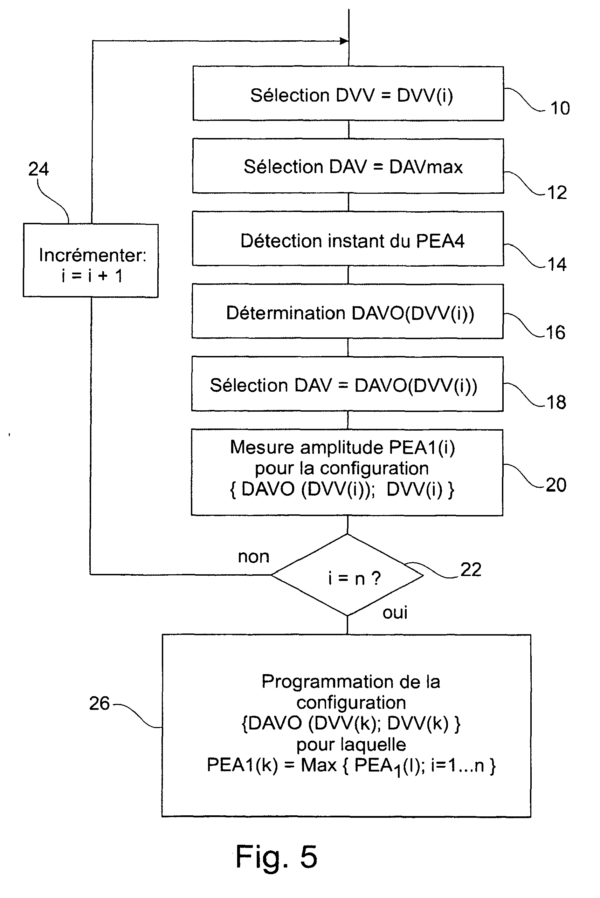

On va maintenant expliciter la séquence de mise en oeuvre de l'invention, en référence aux Figures 4 et Figure 5. La Figure 4 illustre les paramètres du signal EA utilisés, et la Figure 5 indique les diverses étapes successivement exécutées.

Comme on l'a indiqué plus haut, l'invention repose, essentiellement, sur l'analyse concomitante de deux paramètres du signal EA au cours d'un même cycle cardiaque, à savoir :

- un paramètre temporel lié à la composante EA4, pour optimiser le DAV de manière que la stimulation ventriculaire survienne le plus tard possible sans troncature de l'onde A (situation illustrée Figure 3b), et
- un paramètre non-temporel lié à l'ampleur de la composante EA1 (amplitude du pic PEA1, ou énergie de la composante EA1), pour optimiser le DVV de manière que les deux stimulations ventriculaires procurent une contractilité optimale, avec maximisation du volume d'éjection.

Le paramètre temporel lié à la composante EA4 peut être déterminé par une technique telle que celle exposée dans le EP 2 092 885 A1 (ELA Medical), qui décrit diverses manières de produire un marqueur temporel représentatif de la survenue de chacune des diverses composantes EA1 à EA4 du signal EA. Les marqueurs produits par l'algorithme peuvent caractériser l'instant du pic de la composante EA analysée, ou bien un instant de début ou de fin de cette même composante.

En ce qui concerne le paramètre non temporel lié à la composante EA1, dans la suite de la description on considèrera l'amplitude du pic PEA1, c'est-à-dire la valeur maximale crête à crête séparant les deux extrema, positif et négatif, de la composante EA1 du signal d'accélération (valeur repérée $A_{PEA1}$ sur la Figure 4). Ce choix n'est cependant aucunement limitatif, et d'autres paramètres représentatifs de l'ampleur de cette composante peuvent être utilisés, notamment l'énergie du signal EA contenu dans ou partie de cette composante EA1.

On va maintenant expliciter la séquence de mise en oeuvre de l'invention, en référence aux Figures 4 et Figure 5.

Après avoir sélectionné une valeur DVV (i) pour le délai interventriculaire DVV (bloc 10 sur la Figure 5), on sélectionne la valeur maximale $DAV_{max}$ du délai atrioventriculaire (bloc 12), généralement la valeur dite "DAV de base" prévue par la programmation du dispositif. En effet, l'analyse de la composante EA4 implique la programmation d'un DAV long pour être certain d'obtenir une dépolarisation spontanée du ventricule (onde R).

Il convient alors de détecter un instant caractéristique de la composante EA4, par exemple l'instant du pic PEA4 (bloc 14), par l'une des techniques exposées dans le EP précité.

L'instant $t_{PEA4}$ (Figure 4) de ce pic est mesuré en prenant comme origine l'événement ventriculaire immédiatement précédent, spontané (R) ou déclenché (V). On mesure également à partir de cette même origine l'instant $t_{P/A}$ de l'événement auriculaire consécutif, spontané (P) ou déclenché (A). La différence $\Delta t$, entre ces deux instants permet, après addition d'une constante c de compensation, de déterminer (bloc 16) le DAV optimal DAVO, pour cette configuration DVV(i) testée :

$$DAVO(DVV(i)) = t_{PEA4} - t_{P/A} + c$$

Après que le DAV a été établi (bloc 18) à la valeur optimale DAVO déterminée à l'étape précédente, l'amplitude $Ap_{EA1}$ du pic PEA1 est mesurée pour cette même configuration DVV(i) (bloc 20).

Les couples de valeurs {DAVO(DVV(i)), DVV (i)} sont ainsi déterminés pour toutes les configurations de stimulation DVV(i), i = 1... *n* à tester (blocs 22, 24). On obtient ainsi les valeurs de DAV optimales DAVO pour chacune des configurations.

L'étape finale (bloc 26) consiste à sélectionner, parmi tous les couples de valeurs déterminés ainsi, la configuration {DAVO(DVV(k)), DVV(k)} optimale, qui sera celle donnant l'amplitude de PEA1 est la plus élevée.

Éventuellement, pour réduire l'erreur de mesure, il est possible de calculer des valeurs moyennes de DAVO et de $A_{PEA1}$ sur plusieurs cycles, par exemple sur 3 à 5 cycles, de préférence sur 4 cycles.

Par ailleurs, dans la mesure où les paramètres mesurés des composantes EA4 et EA1 dépendent de la valeur du DAV du cycle précédent, la mise en oeuvre de l'algorithme doit prendre en compte la séquence des événements auriculaire-ventriculaire-auriculaire.

À cet effet, dans une variante de l'algorithme de la Figure 5, il est possible de procéder par exemple de la manière suivante :

- programmation d'un DAV long, pour obtenir une contraction ventriculaire spontanée ;
- mesure du paramètre $t_{P/A}$ ;
- programmation d'un DAV plus court que l'intervalle PR (par exemple DAV = PR-40 ms ou DAV = 30 % x PR), pour assurer une capture de la stimulation V sans fusion de la composante EA4 avec les autres composantes du signal (au lieu du DAV long avec contraction spontanée du ventricule, comme dans l'algorithme de la Figure 5) ; puis
- mesure du paramètre $t_{PEA4}$ en configuration de stimulation V (au lieu d'une contraction spontanée du ventricule, comme dans l'algorithme de la Figure 5).

Le DAV optimal DAVO(DVV(i)) est déterminé à partir de ces paramètres au moyen de l'algorithme décrit plus haut.

Une autre variante possible consiste à tester une pluralité de DAV (par exemple DAV = PR - 40 ms, DAV = 50 % x PR et DAV = 60 ms), rechercher la composante EA4 déclenchée par la contraction auriculaire (P/A), et mesurer ensuite le paramètre $t_{PEA4}$ pour en déduire le DAV optimal DAVO.

D'autre part, chez certains patients il n'est pas toujours possible de clairement distinguer le pic PEA4, qui peut se trouver partiellement fusionné avec la composante EA2 précédente (relaxation ventriculaire) ou la composante EA1 suivante (contraction ventriculaire). Dans ce cas, au lieu d'utiliser comme paramètre temporel l'instant du pic PEA4 de la composante EA4, il est possible d'utiliser un autre paramètre temporel, par exemple l'instant de début ou l'instant de fin de la composante EA4, pour calculer le DAV optimal, avec une modification correspondante du facteur de correction c dans la formule donnée plus haut de calcul du DAVO.

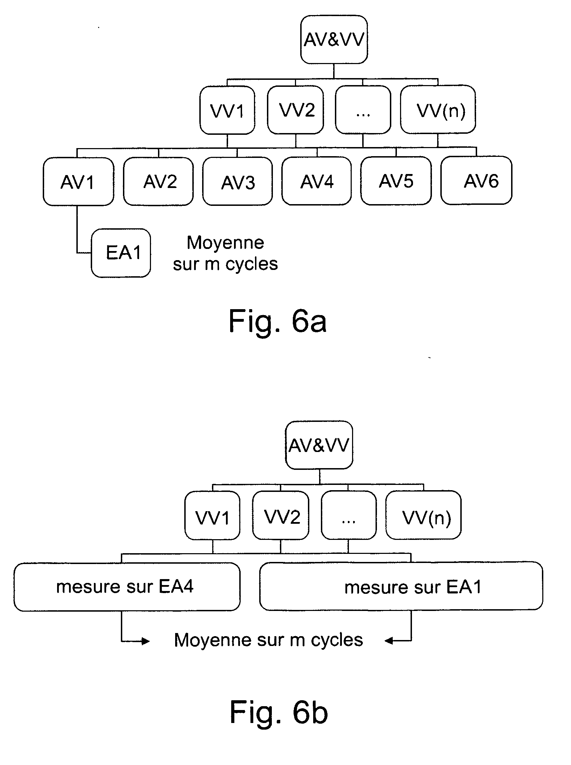

Les Figures 6a et 6b sont des arbres de séquencement illustrant le gain de temps procuré par l'invention pour la détermination du paramétrage optimal du dispositif, par rapport aux techniques antérieures.

La Figure 6a correspond à une méthode de mesure basée sur un calcul de l'aire avec balayage du délai AV, tel que décrite par exemple dans le EP 1 736 203 A1 précité.

L'optimisation des délais AV & VV (premier niveau) implique le test de n configurations VV1... VV(n) (deuxième niveau).

Pour chacune de ces configurations VV(i), le dispositif doit exécuter un balayage du DAV, généralement avec un minimum de 6 valeurs AV1... AV6 (troisième niveau).

Pour chaque valeur AVi du DAV dans chacune des configurations VVi, le dispositif mesure l'amplitude du PEA1 (ou autre paramètre lié à la composante EA1), cette mesure étant moyennée sur m cycles (typiquement, m = 4 cycles).

Avec cette méthode antérieure, la détermination du DVV optimal pour les n configurations VVi testées nécessite donc (n x m x 6) cycles, auxquels il faut ajouter au moins un cycle de stabilisation entre chaque changement du DAV (non compté dans le calcul précédent, pour simplifier). Enfin, la détermination du DVV optimal par la méthode précitée connue est suivie par l'optimisation du DAV optimal dans la configuration optimale (DVVO), qui nécessite (m x 11) cycles. Un total de [(n x m x 6) + (m x 11)] cycles est donc nécessaire pour obtenir le couple optimal {DAVO, DVVO}.

En revanche, dans le cadre de l'invention, comme illustré sur la Figure 6b, pour chaque configuration VVi, le DAV peut être évalué sans balayage, avec mesure concomitante du paramètre temporel lié à la composante EA4 et du paramètre non temporel lié à la composante EA1. Comme expliqué plus haut, cette étape requiert un minimum de deux cycles pour la mesure des deux paramètres EA (instant du PEA4 et amplitude du PEA1, dans l'exemple décrit), auxquels il faut ajouter au moins un cycle de stabilisation entre chaque changement du DVV. Si les mesures sont moyennées sur m cycles, la durée totale du test pour les n configurations VVi sera de (n x m x 2) cycles seulement, donc un gain de temps considérable par rapport à la technique antérieure, et ce avec une qualité d'optimisation au moins égale à ce que l'on pouvait obtenir jusqu'à présent.

**Revendications**

1. Un dispositif médical implantable actif du type prothèse cardiaque de resynchronisation par stimulation biventriculaire, comprenant :

    - des moyens de détection d'événements auriculaires et ventriculaires ;
    - des moyens de stimulation des ventricules droit et gauche ;
    - des moyens de calcul d'un délai atrio-ventriculaire DAV, compté à partir de la détection d'un événement auriculaire spontané (P) ou stimulé (A) et à l'issue duquel une stimulation (V) du ventricule droit est appliquée en l'absence d'événement ventriculaire spontané (R) détecté ;
    - des moyens de calcul d'un délai inter-ventriculaire DVV à appliquer entre les instants respectifs de stimulation des ventricules droit et gauche ; et
    - des moyens de recueil d'un signal d'accélération endocardiaque EA représentatif des mouvements produits par les contractions cycliques des cavités auriculaire et ventriculaire ;

dispositif **caractérisé en ce que** les moyens de calcul du DAV et du DVV comprennent :

- des moyens pour reconnaître et isoler dans le signal EA une composante EA4 correspondant au quatrième pic d'accélération endocardiaque associé à l'activité auriculaire, et à mesurer un paramètre temporel lié à l'intervalle de temps entre la détection d'un événement auriculaire (P/A) et un marqueur lié à la composante EA4 immédiatement consécutive ;
- des moyens pour reconnaître et isoler dans le signal EA une composante EA1 correspondant au premier pic d'accélération endocardiaque associé à la contraction ventriculaire isovolumique, et à mesurer un paramètre non temporel lié à l'amplitude du pic, ou à l'énergie du signal, de la composante EA1 ;
- des moyens pour déterminer un DAV optimal en fonction dudit paramètre temporel lié à la composante EA4, ce DAV optimal étant calculé pour chaque valeur d'une pluralité de valeurs différentes de DVV, de manière à obtenir ainsi une pluralité de couples de valeurs {DAV optimal, DVV} ; et
- des moyens pour sélectionner, parmi ladite pluralité de couples de valeurs {DAV optimal, DVV}, l'un de ces couples de valeurs en fonction dudit paramètre non temporel lié à la composante EA1.

2. Le dispositif de la revendication 1, dans lequel les moyens pour sélectionner l'un des couples de valeurs {DAV optimal, DVV} sont des moyens pour sélectionner celui des couples qui maximise l'amplitude du premier pic d'accélération endocardiaque ou, respectivement, l'énergie de la composante EA1.

3. Le dispositif de la revendication 1, dans lequel les moyens pour déterminer le DAV optimal sont des moyens aptes à calculer ce DAV optimal à partir dudit intervalle de temps, augmenté d'une constante de compensation de délai.

4. Le dispositif de la revendication 1, dans lequel ledit marqueur lié à la composante EA4 est un marqueur de l'instant de survenue du quatrième pic d'accélération endocardiaque.

5. Le dispositif de la revendication 1, dans lequel ledit marqueur lié à la composante EA4 est un marqueur de l'instant de début ou de fin de la composante EA4.

6. Le dispositif de la revendication 1, dans lequel lesdits moyens pour déterminer un DAV optimal sont des moyens aptes, pour chaque valeur de ladite pluralité de valeurs différentes de DVV :

- sur un premier cycle cardiaque, à :

• sélectionner au moins une valeur prédéterminée de DAV ;
• mesurer ledit paramètre temporel lié à la composante EA4 ; et
• déterminer une valeur correspondante de DAV optimal,

- puis sur le cycle cardiaque immédiatement consécutif :

• sélectionner une valeur de DAV correspondant à la valeur de DAV optimal déterminée au cycle précédent ; et
• mesurer ledit paramètre non temporel lié à la composante EA1.

7. Le dispositif de la revendication 6, dans lequel ladite valeur prédéterminée de DAV est une valeur longue choisie de manière à assurer une dépolarisation ventriculaire spontanée consécutive.

8. Le dispositif de la revendication 6, dans lequel une pluralité de valeurs prédéterminées de DAV sont sélectionnées au cours de cycles successifs, et le paramètre temporel lié à la composante EA4 est mesuré avec une valeur de DAV pour laquelle la composante EA4 peut être reconnue et isolée.

**Claims**

1. Active implantable medical device of the cardiac prosthesis type for resynchronization through biventricular stimulation, comprising:

- means for detection of atrial and ventricular events;
- means for stimulation of the right and left ventricles;
- means for calculating an atrioventricular delay DAV, which is counted from the detection of a spontaneous atrial event (P) or stimulated atrial event (A) and at the end of which a stimulation (V) of the right ventricle is applied in the absence of a detected spontaneous ventricular event (R);
- means for calculating an interventricular delay DVV to be applied between the respective instants of stimulation of the right and left ventricles; and
- means for collecting an endocardial acceleration signal EA representative of the movements produced by the cyclical contractions of the atrial and ventricular cavities;

said device being **characterized in that** the means for calculating the DAV and the DVV comprise:

- means for recognizing, and for isolating from the EA signal, a component EA4 corresponding to the fourth endocardial acceleration peak associated with the atrial activity, and for measuring a temporal parameter linked to the time interval between the detection of an atrial event (P/A) and a marker linked to the immediately consecutive component EA4;
- means for recognizing, and for isolating from the EA signal, a component EA1 corresponding to the first endocardial acceleration peak associated with the isovolumetric ventricular contraction, and for measuring a non-temporal parameter linked to the amplitude of the peak, or to the energy of the signal, of the component EA1;
- means for determining an optimal DAV as a function of said temporal parameter linked to the component EA4, this optimal DAV being calculated for each value of a plurality of different values of DVV, in such a way as to thereby obtain a plurality of pairs of values {optimal DAV, DVV}; and
- means for selecting, from said plurality of pairs of values {optimal DAV, DVV}, one of these pairs of values as a function of said non-temporal parameter linked to the component EA1.

2. Device according to Claim 1, in which the means for selecting one of the pairs of values {optimal DAV, DVV} are means for selecting the pair that maximizes the amplitude of the first endocardial acceleration peak or, respectively, the energy of the component EA1.

3. Device according to Claim 1, in which the means for determining the optimal DAV are means suitable for calculating this optimal DAV from said time interval, augmented by a delay compensation constant.

4. Device according to Claim 1, in which said marker linked to the component EA4 is a marker of the instant of occurrence of the fourth endocardial acceleration peak.

5. Device according to Claim 1, in which said marker linked to the component EA4 is a marker of the instant of the start or finish of the component EA4.

6. Device according to Claim 1, in which said means for determining an optimal DAV are means which, for each value of said plurality of different values of DVV, are able:

- on a first cardiac cycle:

• to select at least one predetermined value of DAV;
• to measure said temporal parameter linked to the component EA4; and
• to determine a corresponding value of optimal DAV,

- then, on the immediately consecutive cardiac cycle:

• to select a value of DAV corresponding to the optimal value of DAV determined in the preceding cycle; and
• to measure said non-temporal parameter linked to the component EA1.

7. Device according to Claim 6, in which said predetermined value of DAV is a long value chosen in such a way as to ensure a consecutive spontaneous ventricular depolarization.

8. Device according to Claim 6, in which a plurality of predetermined values of DAV are selected during successive cycles, and the temporal parameter linked to the component EA4 is measured with a value of DAV for which the component EA4 can be recognized and isolated.

**Patentansprüche**

1. Aktive implantierbare medizinische Vorrichtung des Typs Herzprothese für die Resynchronisation durch biventrikuläre Stimulation, mit:

- Mitteln zum Detektieren von atrialen und ventrikulären Ereignissen;
- Mitteln für die Stimulation des rechten und des linken Ventrikels;
- Mitteln zum Berechnen einer atrioventrikulären Verzögerung DAV, die anhand der Detektion eines spontanen (P) oder stimulierten (A) atrialen Ereignisses gezählt wird, wobei an ihrem Ende dann, wenn ein detektiertes spontanes Ventrikelereigniss (R) fehlt, eine Stimulation (V) des rechten Ventrikels angewendet wird;
- Mitteln zum Berechnen einer interventrikulären Verzögerung DVV, die zwischen jeweiligen Zeitpunkten einer Stimulation des rechten und des linken Ventrikels anzuwenden ist; und
- Mitteln zum Sammeln eines endokardialen Beschleunigungssignals EA, das Bewegungen repräsentiert, die durch die zyklischen Kontraktionen der atrialen und der ventrikulären Kammern erzeugt werden;

wobei die Vorrichtung **dadurch gekennzeichnet ist, dass** die Mittel zum Berechnen der DAV und der DVV enthalten:

- Mittel, um in dem Signal EA eine Komponente EA4, die der vierten Spitze der endokardialen Beschleunigung entspricht, die der atrialen Aktivität zugeordnet ist, zu erkennen und zu isolieren und um einen Zeitparameter zu messen, der mit dem Zeitintervall zwischen der Detektion eines atrialen Ereignisses (P/A) und einer Markierung, die mit der unmittelbar folgenden Komponente EA4 in Beziehung steht, in Beziehung steht;

- Mittel, um in dem Signal EA eine Komponente EA1, die der ersten Spitze der endokardialen Beschleunigung entspricht, die der isolvolumetrischen ventrikulären Kontraktion zugeordnet ist, zu erkennen und zu isolieren und um einen nicht zeitlichen Parameter, der mit der Amplitude der Spitze oder mit der Energie des Signals der Komponente EA1 in Beziehung steht, zu messen;

- Mittel, um eine optimale DAV als Funktion des zeitlichen Parameters, der mit der Komponente EA4 in Beziehung steht, zu bestimmen, wobei diese optimale DAV für jeden Wert mehrerer verschiedener Werte von DVV berechnet wird, derart, dass auf diese Weise mehrere Wertepaare {optimale DAV, DVV} erhalten werden; und

- Mittel, um aus den mehreren Wertepaaren {optimale DAV, DVV} eines dieser Wertepaare als Funktion des nicht zeitlichen Parameters, der mit der Komponente EA1 in Beziehung steht, auszuwählen.

2. Vorrichtung nach Anspruch 1, wobei die Mittel für die Auswahl eines der Wertepaare {optimale DAV, DVV} Mittel sind, um jenes der Paare auszuwählen, das die Amplitude der ersten Spitze der endokardialen Beschleunigung bzw. die Energie der Komponente EA1 maximal macht.

3. Vorrichtung nach Anspruch 1, wobei die Mittel zum Bestimmen der optimalen DAV Mittel sind, die diese optimale DAV anhand des Zeitintervalls, erhöht um eine Verzögerungskompensationskonstante, berechnen können.

4. Vorrichtung nach Anspruch 1, wobei die Markierung, die mit der Komponente EA4 in Beziehung steht, eine Markierung des Zeitpunkts des Auftretens der vierten Spitze der endokardialen Beschleunigung ist.

5. Vorrichtung nach Anspruch 1, wobei die Markierung, die mit der Komponente EA4 in Beziehung steht, eine Markierung des Zeitpunkts des Beginns oder des Endes der Komponente EA4 ist.

6. Vorrichtung nach Anspruch 1, wobei die Mittel zum Bestimmen einer optimalen DAV Mittel sind, die für jeden Wert der mehreren verschiedenen Werte der DVV in der Lage sind:

- in einem ersten Herzzyklus:

• wenigstens einen vorgegebenen Wert für DAV auszuwählen;
• den zeitlichen Parameter, der mit der Komponente EA4 in Beziehung steht zu messen; und
• einen entsprechenden Wert der optimalen DAV zu bestimmen,

- dann in jedem unmittelbar folgenden Herzzyklus:

• einen Wert für DAV auszuwählen, der dem im vorhergehenden Zyklus bestimmten Wert der optimalen DAV entspricht; und
• den nicht zeitlichen Parameter, der mit der Komponente EA1 in Beziehung steht, zu messen.

7. Vorrichtung nach Anspruch 6, wobei der vorgegebene Wert für DAV ein langer Wert ist, der so gewählt ist, dass eine aufeinander folgende spontane ventrikuläre Depolarisation sichergestellt ist.

8. Vorrichtung nach Anspruch 6, wobei während aufeinander folgender Zyklen mehrere vorgegebene Werte für DAV ausgewählt werden und der zeitliche Parameter, der mit der Komponente EA4 in Beziehung steht, mit einem Wert für DAV gemessen wird, für den die Komponente EA4 erkannt und isoliert werden kann.

Fig. 1

Fig. 2

Fig. 3

Fig. 4

```
                              ┌────────────────────────────────────┐
                              │     Sélection DVV = DVV(i)          │──── 10
                              └────────────────────────────────────┘
                              ┌────────────────────────────────────┐
                              │     Sélection DAV = DAVmax          │──── 12
              24              └────────────────────────────────────┘
               │             ┌────────────────────────────────────┐
    ┌──────────────────┐     │     Détection instant du PEA4       │──── 14
    │   Incrémenter:   │     └────────────────────────────────────┘
    │   i = i + 1      │     ┌────────────────────────────────────┐
    └──────────────────┘     │   Détermination DAVO(DVV(i))        │──── 16
                              └────────────────────────────────────┘
                              ┌────────────────────────────────────┐
                              │   Sélection DAV = DAVO(DVV(i))      │──── 18
                              └────────────────────────────────────┘
                              ┌────────────────────────────────────┐
                              │     Mesure amplitude PEA1(i)        │
                              │     pour la configuration           │──── 20
                              │   { DAVO (DVV(i));  DVV(i) }         │
                              └────────────────────────────────────┘
         non                          ◇  i = n ?  ◇──── 22
                                           │ oui
                              ┌────────────────────────────────────┐
                              │    Programmation de la              │
           26                 │    configuration                    │
                              │   {DAVO (DVV(k); DVV(k) }            │
                              │    pour laquelle                    │
                              │   PEA1(k) = Max { PEA1(I); i=1...n } │
                              └────────────────────────────────────┘
```

# Fig. 5

Fig. 6a

Fig. 6b

**RÉFÉRENCES CITÉES DANS LA DESCRIPTION**

**Documents brevets cités dans la description**

- US 20050027320 A1
- EP 1108446 A1
- EP 1736203 A1
- EP 0515319 A1
- US 20070179542 A1
- EP 2092885 A1

**Littérature non-brevet citée dans la description**

- **JM Dupuis et al.** Programming Optimal Atrioventricular Delay in Dual Chamber Pacing Using Peak Endocardial Acceleration : Comparison with a Standard Echocardiographic Procedure. *PACE,* 2003, vol. 26, 210-213